# EUROPEAN PATENT SPECIFICATION

(11) **EP 2 144 572 B1**
(45) Date of publication and mention of the grant of the patent: **21.09.2011**
(21) Application number: 07761102.8
(22) Date of filing: 23.04.2007
(51) Int. Cl.: A61C 7/36, A61C 7/02, A61F 5/01

(54) **MANDIBULAR ADVANCEMENT APPLIANCE**
VORRICHTUNG ZUM VORSCHIEBEN DES UNTERKIEFERS
APPAREIL D'AVANCEMENT MANDIBULAIRE

(43) Date of publication of application: 20.01.2010
(73) Proprietor: Distar L.l.c, Albuquerque, NM 87111 (US)
(72) Inventor: MEADE, Thomas, E., Albuquerque, NM 87111 (US)
(74) Representative: Allee, Harriet Eva Charlotta
(86) International application number: PCT/US2007/067195
(87) International publication number: WO 2008/130413

(56) References cited:
- EP-A1- 1 972 311
- DE-U1-202007 007 760
- US-A- 3 618 214
- US-A- 4 505 672
- US-A- 5 066 226
- US-A- 6 055 986
- US-A- 6 109 265
- US-A1- 2007 074 729
- US-B1- 6 572 372

## Description

This invention is related to oral appliances for preventing or alleviating snoring and sleep apnea. The appliance is a removable mandibular advancement device that uses coil springs to pull the lower jaw forward during use.

### BACKGROUND

It is known in the art that an oral appliance that serves to move the mandible forward reduces sleep apnea and snoring of a user. A variety of appliances are available that are removable and advance the mandible. Some of these appliances are adjustable to provide different mandible advancement forces.

One such appliance issued to Frantz et al on Aug. 29, 2000, U.S. Patent No. 6,109,265, uses elastic bands hooked between upper and lower trays to pull the mandible forward. The elastic urethane bands are interchangeable with other bands having varying lengths and/or elasticity to provide different advancement forces on the mandible. This appliance uses rubber bands, which tend to break with use, and requires new rubber bands to be applied to change the tension.

Other such appliances are disclosed in U.S. Patent Nos. 4,505,672 to Kurz, 5,775,219 to Thronton, 6,450,167 to David et al., 5,947,724 to Frantz et al., 6,729,335 to Halstrom, and 5,467,783, 5,682,903, and 6,055,986 to Meade.
US 2007/0074729 A1 relates to a mandibular advancement splint against snoring and sleep apnea. The splint is made of two thermoformable trays designed to envelop the upper and lower arch, and includes an articulated frame having rigid and flexible elements, immersed in the thermoformable flexible material or molded around it. The upper and lower trays are connected by so-called advancement means, e.g. elastic loops, fastened to the trays by hooks. US 3,618,214 A relates to an orhtodontic spring appliance to replace rubber or latex elastic bands for application of corrective intermaxillary forces to teeth. The appliance includes several coaxial coil springs secured together at their ends and having a protective flexible sheath therearound. A pair of eyelets are secured at opposite ends of the assembly for engagement with hooks on orthodontic arch wires, brackets etc.

### SUMMARY

The present invention is a removable, adjustable oral appliance for alleviating snoring and sleep apnea in a user. The appliance has tension springs fitted between ball type hook supports on both sides of an upper teeth conforming tray and extending in tension to one of a plurality of ball type hook supports on the teeth conforming lower tray. These springs provide discrete levels of tension force to the lower jaw to advance the jaw forward.

### BRIEF DESCRIPTION OF THE DRAWINGS

Fig. 1 is a side view of the upper and lower trays with the tension coil springs in place.

Fig. 2 is a side view of a coil spring having a protective plastic sleeve thereon and

Fig. 3 is an exploded view

### DETAILED DESCRIPTION

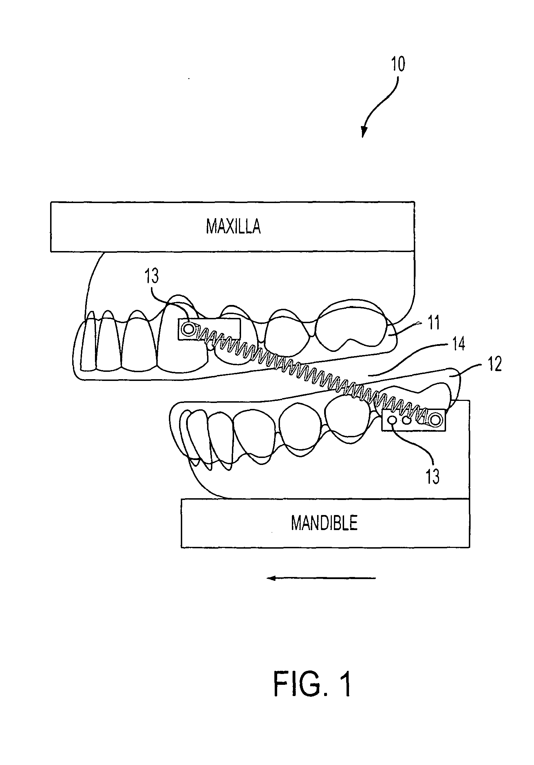

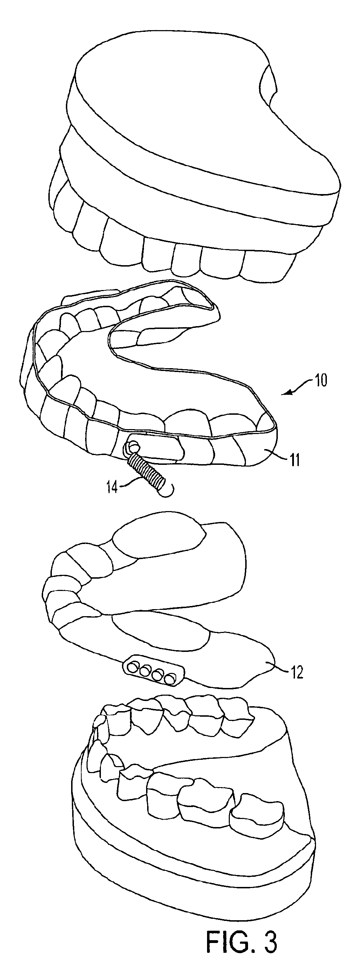

Referring to Figs. 1 and 3 the appliance 10 includes an upper tray 11 that conforms with the user's upper teeth including maxillary dentition soft tissue and palate and includes a ball type hook support 13 on each side of the tray, and a lower tray 12 that conforms to the user's lower teeth including mandibular dentition and soft tissue and that includes a plurality of ball type hook supports 13 on each side of the tray. In one embodiment, there are four ball type hook supports on each side of the lower tray 12. The upper hook support on each side of the upper tray is attached at a forward portion of the tray, and the plurality of hook supports on each side of the lower tray are attached at a rearward portion of the tray. The upper and lower trays are injection molded and made of acrylic plastic. Other materials may be used, as well, which have similar properties to the acrylic plastic. The upper and lower trays may also be laboratory fabricated with the spherical ball attached. The balls may be in injection molded and attached to the upper and lower trays with an adhesive.

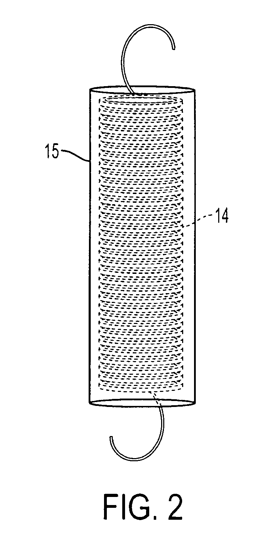

Each of the hook supports 13 have smooth spherical balls and a stem extending therefrom that fits into corresponding holes in the upper and lower trays and are bonded thereto. Each spring 14 shown in Fig. 2 may be made of plastic or stainless steel and has a curved hook at each end. A sleeve 15 as seen in Fig. 2 may be positioned over each spring to guard against injury to a user's gums. The sleeve 15 may be made of plastic or other suitable material.

In use, a doctor would determine the proper strength coil springs to be used and would hook one end of the coil springs to the hook support on each side of the upper tray and hook the other end of the coil springs to one of the plurality of hook supports on each side of the lower tray. The particular hook support used to hook the spring ends on the lower tray would be determined by the doctor to provide a predetermined tension force to each side of the lower tray to advance the mandible forward under the proper tension in the direction of the arrow seen in Fig. 1.

The appliance of the invention provides many advantages over prior art appliances including posterior support for all posterior teeth, complete vertical and lateral freedom of movement of the mandible, no sharp edges that may injure a user, and easy adjustment of the mandible advancement force. Furthermore, the tension springs are less likely to break as compared with elastic bands.

## Claims

1. An oral appliance (10) for use in advancing a user's lower jaw, comprising upper and lower trays (11, 12) that fit to a user's upper and lower teeth, **characterized in that**:
a pair of ball type hook supports (13) are attached one on each side of the forward portion of the upper tray (11),
a plurality of ball type hook supports (13) are attached to each side of the rear portion of the lower tray (12), and
a pair of tension coil springs (14) are provided, wherein each spring (14) is hooked at one end thereof to the hook support (13) at each side of the upper tray (11), and is hooked to one of the plurality of hook supports (13) at each side of the lower tray (12),
whereby the spring force of each coil spring (14) on the lower tray (12) may be adjusted by moving the hooked end on each side of the lower tray (12) to another hook support (13).

2. The oral appliance of claim 1, wherein each hook support (13) comprises a spherical member having a stem extending therefrom, the stem fitting into a corresponding hole in the upper and lower trays (11, 12).

3. The oral appliance of claim 1, further comprising a plastic sleeve (15) positioned over each of the coil springs (13).

4. The oral appliance of claim 1, wherein the plurality of ball type hook supports (13) comprises four hook supports (13) on each side of the lower tray.

5. The oral appliance of claim 1, wherein the coil springs (14) are made of stainless steel.

6. The oral appliance of claim 1, wherein the upper and lower trays (11, 12) are injection molded acrylic members.

## Patentansprüche

1. Mundvorrichtung (10) zur Verwendung beim Vorschieben des Unterkiefers eines Benutzers, umfassend obere und untere Platten (11, 12), die zu den oberen und unteren Zähnen eines Benutzers passen, **dadurch gekennzeichnet, dass**:
ein Paar Kugelanker (13), einer an jeder Seite des vorderen Bereichs der oberen Platte (11), angebracht ist,
eine Mehrzahl von Kugelankern (13) an jeder Seite des hinteren Bereichs der unteren Platte (12) angebracht ist, und
ein Paar von Schraubenzugfedern (14) vorgesehen ist, wobei jede Feder (14) mit einem Ende davon am Kugelanker (13) an jeder Seite der oberen Platte (11) eingehakt ist und an einem der Mehrzahl von Kugelankern (13) an jeder Seite der unteren Platte (12) eingehakt ist,
wodurch die Federspannung jeder Schraubenfeder (14) an der unteren Platte (12) angepasst werden kann, indem das eingehakte Ende an jeder Seite der unteren Platte (12) zu einem anderen Kugelanker (13) verlagert werden kann.

2. Mundvorrichtung nach Anspruch 1, wobei jeder Kugelanker (13) ein kugelförmiges Element umfasst, von dem aus sich eine Stange erstreckt, wobei die Stange in ein entsprechendes Loch in der oberen und unteren Platte (11, 12) passt.

3. Mundvorrichtung nach Anspruch 1, des Weiteren aufweisend eine Kunststoffhülse (15), die über jeder der Schraubenfedern (14) positioniert ist.

4. Mundvorrichtung nach Anspruch 1, wobei die Mehrzahl von Kugelankern (13) vier Kugelanker (13) an jeder Seite der unteren Platte umfasst.

5. Mundvorrichtung nach Anspruch 1, wobei die Schraubenfedern (14) aus rostfreiem Stahl gefertigt sind.

6. Mundvorrichtung nach Anspruch 1, wobei die obere und die untere Platte (11, 12) im Spritzgussverfahren hergestellte Acrylelemente sind.

## Revendications

1. Appareil dentaire (10) destiné à l'avancement d'une mâchoire inférieure d'un utilisateur, comprenant des plateaux inférieur et supérieur (11, 12) qui s'adaptent aux dents supérieures et inférieures d'un utilisateur, **caractérisé en ce** qui :
une paire de supports en crochet de type à bille (13) sont fixés sur chaque côté de la partie avant du plateau supérieur (11),
une pluralité de supports en crochet de type à bille (13) est fixée à chaque côté de la partie arrière du plateau inférieur (12), et
une paire de ressorts hélicoïdaux de tension (14) est fournie, dans laquelle chaque ressort (14) est accroché à une extrémité de celui-ci (13) à chaque côté du plateau supérieur (11), et est accroché à l'une de la pluralité des supports de crochet (13) à chaque côté du plateau inférieur (12),
moyennant quoi la force du ressort de chaque ressort hélicoïdal (14) sur le plateau inférieur (12) peut être ajustée en déplaçant l'extrémité en crochet sur chaque côté du plateau inférieur (12) vers un autre support de crochet (13).

2. Appareil dentaire selon la revendication 1, dans lequel chaque support de crochet (13) comprend un élément sphérique ayant une tige s'étendant à partir de celui-ci, la tige s'adaptant dans un trou correspondant dans les plateaux supérieur et inférieur (11, 12).

3. Appareil dentaire selon la revendication 1, comprenant en outre un manchon en plastique (15) positionné sur chacun des ressorts hélicoïdaux (13).

4. Appareil dentaire selon la revendication 1, dans lequel la pluralité des supports en crochet de type à bille (13) comprend quatre supports de crochet (13) sur chaque côté du plateau inférieur.

5. Appareil dentaire selon la revendication 1, dans lequel les ressorts hélicoïdaux (14) sont composés d'acide inoxydable.

6. Appareil dentaire selon la revendication 1, dans lequel les plateaux supérieur et inférieur (11, 12) sont des éléments en acrylique moulé.
